# EUROPÄISCHE PATENTSCHRIFT

(11) **EP 1 575 600 B1**
(45) Veröffentlichungstag und Bekanntmachung des Hinweises auf die Patenterteilung: **20.02.2008**
(21) Anmeldenummer: 02779426.2
(22) Anmeldetag: 26.09.2002
(51) Int. Cl.: A61K 31/785, A61K 31/14, A61K 9/107, A61P 17/00, A61P 31/04, A61P 31/10, A61P 31/12

(54) **KOSMETISCHE ZUBEREITUNGEN ENTHALTEND POLYHEXAMETHHYLENBIGUANID-HYDROCHLORID UND DISTEARYLDIMETHYLAMMONIUMCHLORID**
COSMETIC PREPARATIONS OF POLYHEXAMETHYLENEBIGUANIDINE HYDROCHLORIDE AND DISTEARYLDIMETHYLAMMONIUM CHLORIDE
PREPARATIONS COSMETIQUES A BASE D'HYDROCHLORURE DE POLYHEXAMETHYLENEBIGUANIDE ET DE CHLORURE DE DISTEARYLDIMETHYLAMMONIUM

(43) Veröffentlichungstag der Anmeldung: 21.09.2005
(73) Patentinhaber: Beiersdorf AG, 20245 Hamburg (DE)
(72) Erfinder: KRÖPKE, Rainer, 22869 Schenefeld (DE); ZELLE, Dagmar, 21640 Bliedersdorf (DE); FILBRY, Alexander, 22459 Hamburg (DE); HAMER, Gunhild, 22455 Hamburg (DE)
(86) Internationale Anmeldenummer: PCT/EP2002/010817
(87) Internationale Veröffentlichungsnummer: WO 2004/032945

(56) Entgegenhaltungen:
- EP-A- 0 788 797
- WO-A-01/35743
- US-A- 5 668 084

## Beschreibung

Die vorliegende Erfindung betrifft Wirkstoffkombinationen aus Polyhexamethylenbiguanid-Hydrochlorid und Distearyldimethylammoniumchlorid sowie die Verwendung solcher Wirkstoffkombinationen als gegen Bakterien, Mycota und Viren wirksame Substanzen, insbesondere zur Konservierung kosmetischer und dermatologischer Emulsionen. In besonderen Ausführungsformen betrifft die vorliegende Erfindung Zubereitungen, bevorzugt kosmetische und dermatologische Zubereitungen, solche Substanzen enthaltend.

Der gesunde warmblütige Organismus, insbesondere die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bakterien gehören zu den prokaryontischen Einzellem. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Gegen Bakterien wirksame Substanzen sind seit geraumer Zeit bekannt. Allerdings sind viele dieser antibakteriellen Wirkstoffe nicht für alle medizinischen, schon gar nicht kosmetischen Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung war also, den Stand der Technik zu bereichern und Substanzen zur Verfügung zu stellen, welche gegen grampositive und/oder gramnegative Bakterien wirksam sind, ohne daß mit der Anwendung der Substanzen eine unvertretbare Beeinträchtigung der Gesundheit des Anwenders verbunden wäre.

Gramnegative Keime sind beispielsweise Escherichia coli, Pseudomonas-Arten sowie Enterobacteriaceen, wie etwa Citrobacter freundii.

Auch grampositive Keime spielen in Kosmetik und Dermatologie eine Rolle. Bei unreiner Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Staphylococcus aureus: Dieser ist u. a. für die Krankheitsbilder Impetigo follicularis, Furunkulose, Abszesse, Sepsis, Pemphigus neonatorum, toxisches Schocksyndrom, Lebensmittelvergiftung verantwortlich. Darüber hinaus haben Untersuchungen ergeben, daß Neurodermatiker zu einem hohen Prozentsatz (mehr als 90 % der Betroffenen) einen teilweisen extrem erhöhten Staphylococcus aureus-Gehalt auf der Haut haben.

Pilze, auch Fungi, Mycota oder Mycobionten genannt, zählen im Gegensatz zu den Bakterien zu den Eucaryonten. Eucaryonten sind Lebewesen, deren Zellen (Eucyten) im Gegensatz zu denen der sogenannten Procaryonten (Procyten) über einen durch Kemhülle und Kemmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z. B. Epidermophytie, Favus, Mikrosporie, Trichophytie), Hefemycosen (z. B. Pityriasis und andere Pityrosporum-bedingte Mycosen, Candida-Infektionen, Blastomycose, Busse-Buschke-Krankheit, Torulose, Piedra alba, Torulopsidose, Trichosporose), Schimmelmycosen (z. B. Aspergillose, Kephalosporidose, Phycomycose und Skopulariopsidose), Systemmycosen (z. B. Chromomycose, Coccidiomycose, Histoplasmose).

Zu den pathogenen und fakultativ pathogenen Keimen gehören beispielsweise aus der Gruppe der Hefen Candida-Arten (z. B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung von Pityrosporum ovale an der Entstehung von Psoriasis wird von der Fachwelt diskutiert.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche.

Ferner sind Superinfektionen der Haut durch Pilze und Bakterien nicht selten.

Bei bestehendem Primärinfekt, d. h. wenn bei normaler Keimbesiedelung der Haut eine Neuinfektion mit hohen Keimzahlen eines oder mehrerer oft physiologischer Erreger - beispielsweise Staphylokokken - oft aber auch unphysiologischer Erreger - beispielsweise Candida albicans - eintritt, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich - in Abhängigkeit vom betreffenden Keim - in günstig verlaufenden Fällen in unangenehmen Hauterscheinungen (Juckreiz, unschönes äußeres Erscheinungsbild) äußern, In ungünstig verlaufenden Fällen können sie aber zu großflächiger Zerstörung der Haut führen, im schlimmsten Falle sogar im Tode des Patienten gipfeln.

Derartige Superinfektionen werden bei einer Vielzahl dermatologischer Erkrankungen, z. B. atopischem Ekzem, Neurodermitis, Akne, seborrhoischer Dermatitis oder Psoriasis beobachtet. Auch viele medizinische und therapeutische Maßnahmen, z. B. die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z. B. Umweltverschmutzung, Smog), fördern das Auftreten von Superinfektionen der äußeren und inneren Organe, insbesondere der Haut und der Schleimhäute.

Zwar ist es im Einzelfall ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nennenswerte Einbußen erleidet.

US-A-5 668 084 beschreibt die Verhinderung der Bildung von Ablagerungen an der Grenzfläche zwischen Wasser und Luft durch Polyhexamethylenbiguanidinhydrochlorid als antimikrobiell wirksame Substanz in Kombination mit Tensiden, wie Distearyldimethylammoniumchlorid.

WO-A-01/ 35743 offenbart biozide Verbindungen, die Polyhexamethylenbiguanid-hydrochlorid und ein quartäres Ammoniumsalz mit einer Wirkung gegen grampositive und gramnegative Bakterien enthalten, wobei Distearyldimethylammoniumchlorid nicht ausdrücklich offenbart ist.

Die Verwendung von Polyhexamethylenbiguanid zusammen mit einem Tensid zur Behandlung von bakteriellen Infektionen ist außerdem aus EP-A-0 788 797 bekannt.

Im Gegensatz zu den prokaryotischen und eukaryotischen zellulären Organismen sind Viren biologische Strukturen, welche zur Biosynthese eine Wirtszelle benötigen. Extrazelluläre Viren (auch "Virionen" genannt) bestehen aus einer ein- oder doppelsträngigen Nukleinsäuresequenz (DNS oder RNS) und einem Proteinmantel (dapsid genannt), gegebenenfalls einer zusätzlichen lipidhaltigen Hülle (Envelope) umgeben. Die Gesamtheit aus Nukleinsäure und Capsid wird auch Nucleocapsid genannt. Die Klassifikation der Viren erfolgte klassisch nach klinischen Kriterien, heutzutage allerdings zumeist nach ihrer Struktur, ihrer Morphologie, insbesondere aber nach der Nukleinsäuresequenz.

Medizinisch wichtige Virengattungen sind beispielsweise Influenzaviren (Familie der Orthomyxoviridae), Lyssaviren (z. B. Tollwut, Familie der Rhabdoviren) Enteroviren (z. B. Hepatitis-A, Familie der Picomaviridae), Hepadnaviren (z. B. Hepatitis-B, Familie der Hepadnaviridae).

Viruzide, also Viren abtötende Substanzen im eigentlichen Sinne gibt es nicht, da Viren nicht über eigenen Stoffwechsel verfügen. Pharmakologische Eingriffe ohne Schädigung der nicht befallenen Zellen sind jedenfalls schwierig. Mögliche Wirkmechanismen im Kampf gegen Viren sind in erster Linie die Störung deren Replikation, z. B. durch Blockieren der für die Replikation wichtigen Enzyme, die in der Wirtszelle vorliegen. Ferner kann das Freisetzen der viralen Nukleinsäuren in die Wirtszelle verhindert werden. Im Rahmen der hiermit vorgelegten Offenbarung wird unter Begriffen wie "antiviral" oder "gegen Viren wirksam", "viruzid" oder ähnlichen die Eigenschaft einer Substanz verstanden, einen ein- oder mehrzelligen Organismus vor schädlichen Folgen einer Virusinfektion, sei es prophylaktisch oder therapeutisch, zu schützen, ungeachtet dessen, was der tatsächliche Wirkmechanismus der Substanz im Einzelfalle sei.

Dem Stand der Technik mangelt es an gegen Viren wirksamen Substanzen, welche zudem den Wirtsorganismus nicht oder nicht in vertretbarem Maße schädigen.

Eine weitere Aufgabe der vorliegenden Erfindung war daher, Substanzen zu finden, welche wirksam einen ein- oder mehrzelligen Organismus vor schädlichen Folgen einer Virusinfektion, sei es prophylaktisch oder therapeutisch, zu schützen.

Konservierungsmittel sind antimikrobielle Substanzen, die einem Produkt (Nahrungs- oder Genußmittel, pharmazeutische, kosmetische oder auch chemisch-technische Zubereitungen) beim Herstellungsprozeß in geringen Mengen (gewöhnlich je nach Produkt zwischen ca. 0,0005 % und 1 % Aktivgehalt) zugesetzt werden. Konservierungsmittel sollen Produkte während der Herstellung, der Lagerung und des Gebrauchs vor Verunreinigungen durch Mikroorganismen insbesondere vor den mikrobiell bedingten nachteiligen Veränderungen schützen.

An ein Konservierungsmittel werden grundsätzlich nachfolgende Forderungen gestellt: Es muß ausreichend antimikrobiell wirksam, technologisch anwendbar und gesundheitlich unbedenklich sein. Die gesundheitliche Unbedenklichkeit muß auch die fertige Zubereitung - das Handelsprodukt - erfüllen. Dabei ist zu berücksichtigen, daß Mikroorganismen z. B. in kosmetischen Mitteln primär produktionsbedingt vorhanden sein oder sekundär durch den Verbraucher in das kosmetische Mittel gelangen können.

Es muß daher gewährleistet sein, daß das Fertigprodukt auch über den gesamten Verbrauchszeitraum sicher ist.

In der Lebensmitteltechnologie zugelassene Konservierungsmittel - wie beispielsweise die im folgenden aufgelisteten - können vorteilhaft auch für die Herstellung von pharmazeutischen oder kosmetischen Produkten verwendet werden.

| | | | |
|---|---|---|---|
| E 200 | Sorbinsäure | E 227 | Calciumhydrogensulfit |
| E 201 | Natriumsorbat | E 228 | Kaliumhydrogensulfit) |
| E 202 | Kaliumsorbat | E 230 | Biphenyl (Diphenyl) |
| E 203 | Calciumsorbat | E 231 | Orthophenylphenol |
| E 210 | Benzoesäure | E 232 | Natriumorthophenylphenolat |
| E 211 | Natriumbenzoat | E 233 | Thiabendazol |
| E 212 | Kaliumbenzoat | E 235 | Natamycin |
| E 213 | Calciumbenzoat | E 236 | Ameisensäure |
| E 214 | p-Hydroxybenzoesäureethylester | E 237 | Natriumformiat |
| E 215 | p-Hydroxybenzoesäureethylester-Na-Salz | E 238 | Calciumformiat |
| E 216 | p-Hydroxybenzoesäure-n-propytester | E 239 | Hexamethylentetramin |
| E 217 | p-Hydroxybenzoesäure-n-propylester-Na-Salz | E 249 | Kaliumnitrit |
| E 218 | p-Hydroxybenzoesäuremethylester | E 250 | Natriumnitrit |
| E 219 | p-Hydroxybenzoesäuremethylester-Na-Salz | E 251 | Natriumnitrat |
| E 220 | Schwefeldioxid | E 252 | Kaliumnitrat |
| E 221 | Natriumsulfit | E 280 | Propionsäure |
| E 222 | Natriumyhdrogensulfit | E 281 | Natriumpropionat |
| E 223 | Natriumdisutfit | E 282 | Calciumpropionat |
| E 224 | Kaliumdisulfit | E 283 | Kaliumpropionat |
| E 226 | Calciumsulfit | E 290 | Kohlendioxid |

Ferner sind in der Kosmetik gebräuchliche Konservierungsmittel oder Konservierungshilfsstoffe Dibromdicyanobutan (2-Brom-2-brommethylglutarodinitril), 3-lod-2-propinyl-butylcarbamat, 2-Brom-2-nitro-propan-1,3-diol, Imidazolidinylharnstoff, 5-Chlor-2-methyl-4-isothiazolin-3-on, 2-Chloracetamid, Benzalkoniumchlorid, Benzylalkohol.

Ferner wurden Phenylhydroxyalkylether, insbesondere die unter der Bezeichnung Phenoxyethanol bekannte Verbindung aufgrund ihrer bakteriziden und fungiziden Wirkungen auf eine Anzahl von Mikroorganismen als Konservierungsmittel eingesetzt.

Auch Polyhexamethylenbiguanid-Hydrochlorid ist als Konservierungsmittel für O/W- und W/O-Emulsionen an sich bekannt. Polyhexamethylenbiguanid-Hydrochlorid mit der CTFA-Bezeichnung "Polyaminopropyl Biguanide" (CAS-Nr. 28757-47-3) zeichnet sich durch die folgende Strukturformel aus 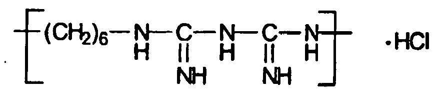 und ist unter Handelsbezeichnung Arlagard® E bei Zeneca Biocides erhältlich.

Arlagard® E ist eine farblose bis schwach gelblich gefärbte, geruchfreie, wäßrige kationische Lösung mit breitem, antimikrobiellen Spektrum (wirksam gegen grampositive, gramnegative Bakterien sowie gegen Hefen und Pilze).

Die meisten für eine Konservierung vorgeschlagenen bzw. vorgesehenen Konservierungsmittel wirken bakteriostatisch und fungistatisch, gelegentlich auch bakterizid und fungizid; sie sollen geruch- und geschmacklos und in den zur Anwendung kommenden Dosen nach Möglichkeit löslich, nicht toxisch, hautverträglich und ausreichend wirksam sein.

Die Konservierungsmittel müssen, um wirksam zu sein, in dem zu konservierenden Roh- oder Hilfsstoff gelöst sein. Da die meisten Konservierungsmittel besser fett- als wasserlöslich sind, muß damit gerechnet werden, daß z. B. in einer Emulsion, deren wäßrige Phase konserviert werden soll, das in die wäßrige Phase eingearbeitete Konservierungsmittel im Verlauf der Lagerung in die Fettphase auswandert und damit die Konservierung der wäßrigen Phase in Frage gestellt ist. Dies stellt insbesondere für die Konservierung von Öl-in-Wasser-Formulierungen ein Problem dar.

Für ein kosmetisches Präparat braucht zwar in der Regel keine Sterilität gefordert zu werden, es muß aber frei von pathogenen Keimen sein und vor mikrobiell bedingten Veränderungen geschützt werden.

Man sollte berücksichtigen, daß die verschiedensten Emulsionstypen, wäßrige Lösungen, Suspensionen usw. eine unterschiedliche Konservierung brauchen, daß die konservierende Wirkung einzelner Konservierungsmittel von der Zusammensetzung und den physikalischen Eigenschaften der zu konservierenden Zubereitung abhängig ist, daß mit Interaktionen zwischen dem Konservierungsmittel, den Wirk- und Hilfsstoffen zu rechnen ist, daß verschiedene Wirk- oder Hilfsstoffe Konservierungsmittel adsorbieren und damit möglicherweise inaktivieren können, daß insbesondere in der Zubereitung enthaltene Hydrokolloide konzentrationsabhängig Konservierungsmittel in ihrer antimikrobiellen Aktivität behindern können und daß schließlich - wiederum in Abhängigkeit von der Konzentration und dem Typ des Konservierungsmittels - das Stratum corneum das Konservierungsmittel adsorbiert und das Konservierungsmittel dann möglicherweise zur Permeation und Absorption kommt.

Es wurde überraschend gefunden, und darin liegt die Lösung aller dieser Aufgaben, daß Wirkstoffkombinationen aus Polyhexamethylenbiguanid-Hydrochlorid und Distearyldimethylammoniumchlorid sowie die Verwendung solcher Wirkstoffkombinationen als antibakterielle, antimycotische oder antivirale Wirkstoffe, den Nachteilen des Standes der Technik abhilft.

Die erfindungsgemäßen Wirkstoffkombinationen wirken erstaunlicherweise in synergistischer Weise, also überadditiv in bezug auf die Einzelkomponenten. Dies war insbesondere deswegen nicht zu erwarten, da Distearyldimethylammoniumchlorid selbst keinerlei antimikrobielle Wirkung zeigt.

Distearyldimethylammöniumchlorid (auch: Dimethyldioctadecylammoniumchlorid, Quaternium-5) mit der CTFA-Bezeichnung "Distearyldimonium Chloride" ist ein quaternäres Ammoniumsalz, welches sich durch die folgende Strukturformel auszeichnet:

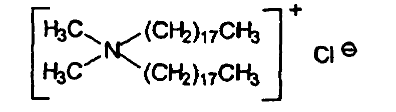

Ganz besonders vorteilhaft ist die Verwendung der erfindungsgemäßen Wirkstoffkombinationen als Konservierungssystem für kosmetische oder dermatologische ÖI-in-Wasser Formulierungen, insbesondere für kosmetische oder dermatologische O/W-Emulsionen.

Es hat sich überraschend herausgestellt, daß die erfindungsgemäß verwendeten Wirkstoffkombinationen - und zwar in überadditiver Weise - das Wachstum von grampositiven und gramnegativen Bakterien, Mycobionten sowie Viren verhindern.

Insbesondere sind die erfindungsgemäß verwendeten Wirkstoffkombinationen befähigt, daß Wachstum von grampositiven Bakterien, insbesondere der Staphylococcus-Arten, namentlich Staphylococcus areus, zu verhindern.

Ferner war erstaunlich, daß die erfindungsgemäß verwendeten Wirkstoffkombinationen besonders gut wirksam sind gegen Neurodermitis und daß sie das Entstehen von typischen Rezidiven der Neurodermitis vermindern. Eine bevorzugte Ausführungsform der vorliegenden Erfindung sind mithin gegen Neurodermitis anzuwendende Formulierungen.

Schließlich hat sich herausgestellt, daß die erfindungsgemäß verwendeten Wirkstoffkombinationen den Verderb organischer Substanz, insbesondere kosmetischer und dermatologischer Zubereitungen, durch den Befall mit grampositiven und gramnegativen Bakterien, Mycobionten und Viren verhindern können, wenn sie diesen Zubereitungen zugesetzt werden.

Erfindungsgemäß sind somit auch ein Verfahren zur Bekämpfung von Mycobionten, dadurch gekennzeichnet, daß die erfindungsgemäß verwendeten Wirkstoffkombinationen gegebenenfalls in einem geeigneten kosmetischen oder dermatologischen Träger, mit dem durch Mycobionten kontaminierten Bereich in Kontakt gebracht werden, sowie ein Verfahren zum Schutz organischer Produkte vor dem Befall mit Mycobionten, dadurch gekennzeichnet, daß diesen organischen Produkten die erfindungsgemäß verwendeten Wirkstoffkombinationen in wirksamer Menge zugegeben werden.

Der Stand der Technik lieferte folglich nicht den geringsten Hinweis auf die erfindungsgemäße Verwendung als antimycotisches Wirkprinzip.

Erfindungsgemäß werden die Wirkstoffkombinationen bevorzugt in kosmetischen oder dermatologischen Zusammensetzungen eingesetzt einem Gehalt von 0,0005 bis 50,0 Gew.-%, insbesondere 0.01 bis 20,0 Gew.-%, bezogen auf das Gesamtgewicht der Zusammensetzung bevorzugt sind. Vorteilhaft enthalten die Zusammensetzungen 0,02 bis 10,0 Gew.-%, besonders bevorzugt 0,02 bis 5,0 Gew.-% an den erfindungsgemäß verwendeten Wirkstoffen, ganz besonders vorteilhaft 0,5 bis 3,0 Gew.-%, jeweils bezogen auf das Gesamtgewicht der Zusammensetzung.

Die erfindungsgemäß verwendeten Wirkstoffkombinationen lassen sich ohne Schwierigkeiten in gängige kosmetische oder dermatologische Formulierungen einarbeiten, vorteilhaft in Pumpsprays, Aerosolsprays, Crèmes, Salben, Tinkturen, Lotionen, Nagelpflegeprodukte (z. B. Nagellacke, Nagellackentferner, Nagelbalsame) und dergleichen.

Als Emulsionen vorliegende Zubereitungen gemäß der vorliegenden Erfindung enthalten einen oder mehrere O/W-Emulgatoren sowie ggf. weitere Co-Emulgatoren. Sollen die Emulsionen weitere O/W-Emulgatoren enthalten, welche neben Distearyldimethylammoniumchlorid in der Zubereitung vorliegen können, so können diese (eine oder mehrere Verbindungen) beispielsweise vorteilhaft gewählt werden aus der Gruppe der polyethoxylierten und/oder polypropoxylierten Emulgatoren.

Der oder die Co-Emulgatoren werden vorteilhaft aus der Gruppe der Fettalkohole mit 14 bis 22 Kohlenstoffatomen, insbesondere aus der Gruppe der Fettalkohole mit 16 bis 18 Kohlenstoffatomen wie Stearylalkohol, Cetylalkohol, Cetylstearylalkohol (Cetearylalkohol) gewählt.

Es ist auch möglich und gegebenenfalls vorteilhaft, die erfindungsgemäß verwendeten Wirkstoffkombinationen mit anderen Wirkstoffen zu kombinieren, beispielsweise mit anderen antimikrobiell, antimycotisch bzw. antiviral wirksamen Stoffen.

Werden die erfindungsgemäßen Wirkstoffkombinationen als Wirkstoffe zur Konservierung organischen Materials eingesetzt, so können auch vorteilhaft zusätzlich ein anderes oder mehrere andere Konservierungsmittel gewählt aus den Substanzen mit den E-Nummem von E-200 bis E-299 eingesetzt werden und/oder ein anderes oder mehrere andere Konservierungsmittel gewählt aus den Substanzen Dibromdicyanobutan (2-Brom-2-brommethylglutarodinitril), Phenoxyethanol, 3-lod-2-propinylbutylcarbamat, 2-Brom-2-nitropropan-1,3-diol, lmidazolidinylharnstoff, 5-Chlor-2-methyl-4-isothiazolin-3-on, 2-Chloracetamid, Benzalkoniumchlorid, Benzylalkohol.

Es ist möglich, wenngleich nicht notwendig, die erfindungsgemäßen Zusammensetzungen abzupuffern. Vorteilhaft ist ein pH-Bereich von 4,5 bis 7,5. Besonders günstig ist es, den pH-Wert in einem Bereich von 5,0 bis 6,5 zu wählen.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die erfindungsgemäßen kosmetischen und/oder dermatologischen Formulierungen in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z. B. üblicherweise für diesen Typ von Zubereitungen eingesetzt werden. So können sie z. B. eine Lösung, eine Emulsion vom Typ Wasser-in-ÖI (W/O) oder vom Typ ÖI-in-Wasser (O/W), oder eine multiple Emulsionen, beispielsweise vom Typ Wasser-in-ÖI-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z. B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate sowie D-Biotin, natürliche und/oder synthetische Isoflavonoide, alpha-Glucosylrutin, Panthenol, Aloe Vera.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Vorteilhafte weitere Wirkstoffe sind natürliche Wirkstoffe und/oder deren Derivate, wie z. B. Phytoen, Carnitin, Camosin, Kreatin, Taurin und/oder β-Alanin.

Die erfindungsgemäßen kosmetischen Zubereitungen können kosmetische Hilfsstoffe enthalten, wie sie üblicherweise in solchen Zubereitungen verwendet werden, z. B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, Dihydroxyaceton, organische Lösungsmittel oder Silikonderivate.

Vorteilhaft können erfindungsgemäße Zubereitungen außerdem Substanzen enthalten, die UV-Strahlung im UVB-Bereich absorbieren, wobei die Gesamtmenge der Filtersubstanzen z. B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwer lösliche oder unlösliche Metallverbindungen, insbesondere Oxide des Titans (TiO₂), Zinks (ZnO), Eisens (z. B. Fe₂O₃). Zirkoniums (ZrO₂), Siliciums (SiO₂), Mangans (z. B. MnO). Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₈, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Vorteilhafte weitere UV-Filtersubstanzen im Sinne der vorliegenden Erfindung sind sutfonierte, wasserlösliche UV-Filter, wie z. B.
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCl Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bomylidenmethyl-10-sulfonsäure) bezeichnet wird. Benzol-1,4-di(2-oxo-3-bomylidenmethyl-10-sulfonsäure) hat die INCl-Bezeichnung Terephtalidene Dicampher Sulfonsäure (CAS.-Nr.: 90457-82-2) und ist beispielsweise unter dem Handetsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bomylidenmethyl)sulfonsäure und deren Salze.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist;
- Dioctylbutylamidotriazon (INCI: Dioctylbutamidotriazone), welches unter der Handelsbezeichnung UVASORB HEB bei Sigma 3V erhältlich ist;
- 4,4',4"-(1,3,5-Triazin-2,4,6-triyltriimino)-tris-benzoäsäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVI-NUL® T 150 vertrieben wird.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

Vorteilhafte wasserlösliche Filtersubstanzen sind z. B.:
Sulfonsäure-Derivate des 3-Benrylidencamphers, wie z. B. 4-(2-Oxo-3-bornylidenmethyl)benzolsulfonsäure, 2-Methyl-5-(2-oxo-3-bomylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist.

Die Liste der genannten UV-Filter, die im Sinne der vorliegenden Erfindung eingesetzt werden können, soll selbstverständlich nicht limitierend sein.

Erfindungsgemäße Zubereitungen können, zumal wenn kristalline oder mikrokristalline Festkörper, beispielsweise anorganische Mikropigmente in die erfindungsgemäßen Zubereitungen eingearbeitet werden sollen, auch nichtionische und/oder amphotere Tenside enthalten. Tenside sind amphiphile Stoffe, die organische, unpolare Substanzen in Wasser lösen können.

Tenside werden im allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wäßriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wäßriger Lösung bilden sie im sauren oder neutralen Milieu positiv geladene organische Ionen. Amphotere Tenside enthalten sowohl anionische als auch kationische Gruppen und verhalten sich demnach in wäßriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z. B. Cr⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z. B. Na⁺ |

Typisch für nicht-ionische Tenside sind Polyether-Ketten. Nicht-ionische Tenside bilden in wäßrigem Medium keine Ionen.

### A. Anionische Tenside

Es ist insbesondere vorteilhaft im Sinne der vorliegenden Erfindung, wenn Zubereitungen enthaltend die erfindungsgemäß verwendeten Wirkstoffkombinationen keine anionischen Tenside enthalten, um eine spontane Salzbildung und damit Inaktivierung der Wirkstoffe zu vermeiden.

### B. Kationische Tenside

Vorteilhaft zu verwendende kationische Tenside sind
1. Alkylamine,
2. Alkylimidazole,
3. Ethoxylierte Amine und
4. Quaternäre Tenside.
5. Esterquats

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte*l* propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7. Polyglycerinester, Diglycerinester, Monoglycerinester
8. Methylglucosester, Ester von Hydroxysäuren

Vorteilhaft ist ferner die Verwendung einer Kombination von kationischen und/oder amphoteren Tensiden mit einem oder mehreren nicht-ionischen Tensiden.

Die oberflächenaktive Substanz kann in einer Konzentration zwischen 1 und 95 Gew.-% in den erfindungsgemäßen Zubereitungen vorliegen, bezogen auf das Gesamtgewicht der Zubereitungen.

Die Lipidphase der erfindungsgemäßen kosmetischen oder dermatologischen Emulsionen kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (z. B. Decamethylcyclopentasiloxan) als erfindungsgemäß zu verwendendes Silikonöl eingesetzt. Aber auch andere Silikonöle sind vorteilhaft im Sinne der vorliegenden Erfindung zu verwenden, beispielsweise Undecamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose. Die folgenden Beispiele sollen die Erfindung erläutern. Die Zahlenangaben beziehen sich auf Gew.-%, sofern nichts anderes angegeben ist.

### Beispiele:

### O/W-Emulsionen

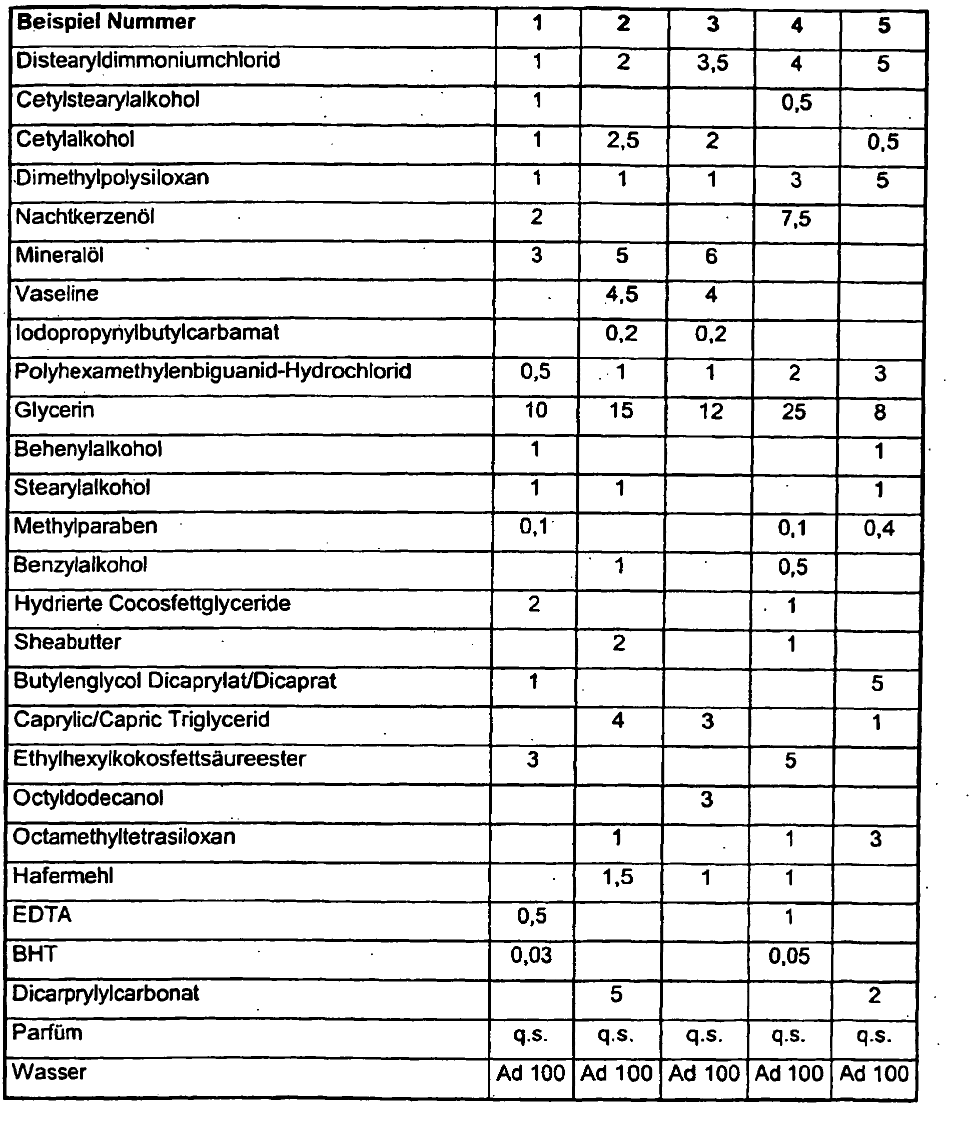

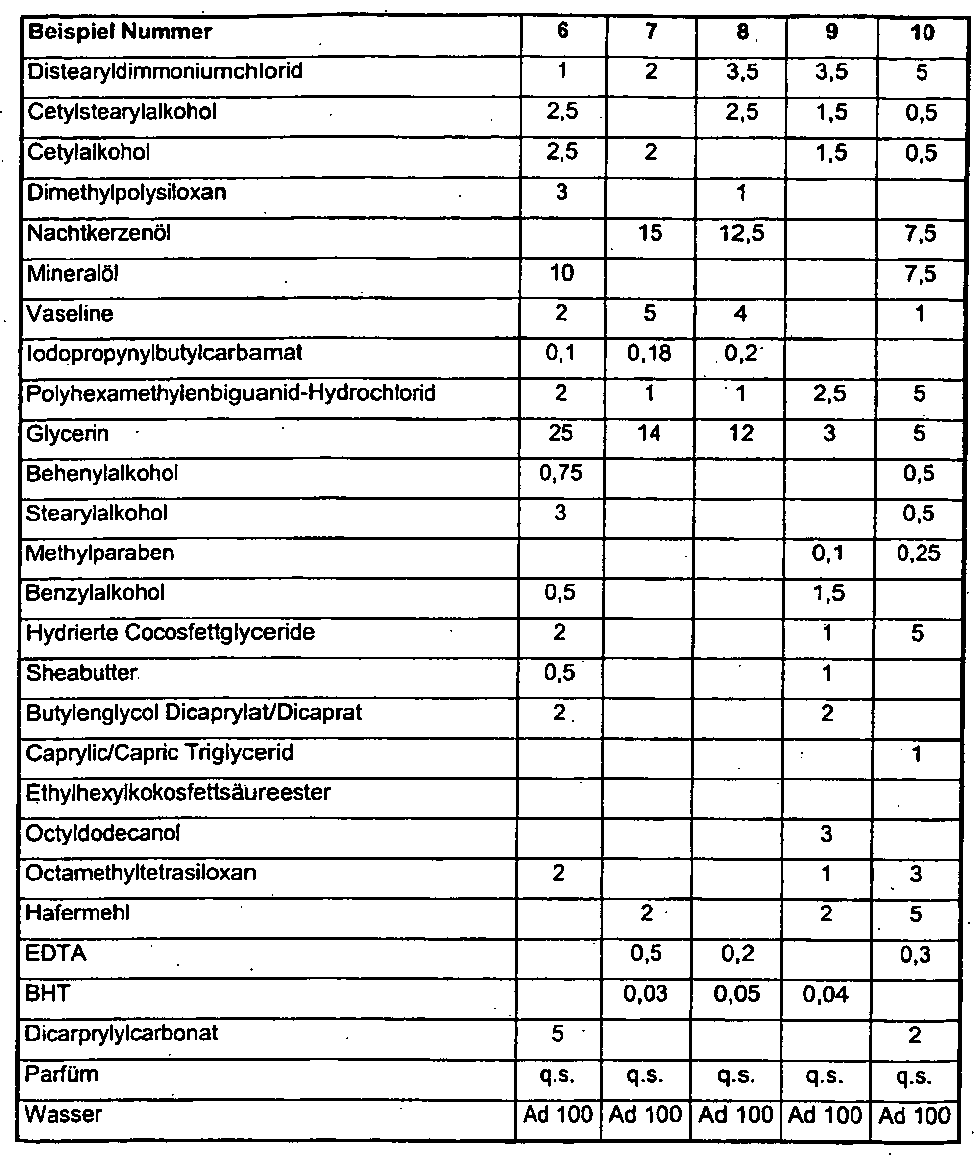

## Patentansprüche

1. Kosmetische Zubereitungen, **dadurch gekennzeichnet, dass** sie in Form einer O/W-Emulsion vorliegen und eine Wirkstoffkombination aus Polyhexamethylenbiguanid-Hydrochlorid und Distearyldimethylammoniumchlorid enthalten.

2. Kosmetische Zubereitungen nach Anspruch 1 **dadurch gekennzeichnet, dass** sie Wirkstoffkombinationen aus Polyhexamethylenbiguanid-Hydrochlorid und Distearyldimethylammoniumchlorid in einem Konzentrationsbereich von 0,0005 bis 50,0 Gew.-%, insbesondere 0,01 bis 20,0 Gew.-%, bezogen auf das Gesamtgewicht der Zusammensetzung enthalten.

3. Kosmetische Zubereitungen nach Anspruch 1 und/oder 2 **dadurch gekennzeichnet, dass** die Wirkstoffe Polyhexamethylenbiguanid-Hydrochlorid und Distearyldimethylammoniumchlorid in einem Konzentrationsbereich von 0,02 bis 10,0 Gew.-%, bevorzugt 0,02 bis 5,0 Gew.-% und insbesondere bevorzugt 0,5 bis 3,0 Gew.-%, bezogen auf das Gesamtgewicht der Zusammensetzung enthalten sind.

4. Kosmetische Zubereitungen nach einem oder mehreren der vorstehenden Ansprüche **dadurch gekennzeichnet, dass** die kosmetischen Zubereitungen einen oder mehrere O/W-Emulgatoren enthalten.

5. Kosmetische Zubereitungen nach einem oder mehreren der vorstehenden Ansprüche **dadurch gekennzeichnet, dass** die kosmetischen Zubereitungen einen oder mehrere Co-Emulgatoren enthalten.

6. Kosmetische Zubereitungen nach Anspruch 4 **dadurch gekennzeichnet, dass** der oder die O/W-Emulgator/en aus der Gruppe der polyethoxylierten und/oder polypropoxylierten Emulgatoren ausgewählt wird/werden.

7. Kosmetische Zubereitungen nach Anspruch 5 **dadurch gekennzeichnet, dass** der oder die Co-Emulgator/en aus der Gruppe der Fettalkohole mit 14 bis 22 Kohlenstoffatomen ausgewählt wird/werden.

8. Kosmetische Zubereitungen nach Anspruch 5 und/oder 7 **dadurch gekennzeichnet, dass** der oder die Co-Emulgator/en aus der Gruppe der Fettalkohole mit 16 bis 18 Kohlenstoffatomen wie Stearylalkohol, Cetylalkohol, Cetylstearylalkohol (Cetearylalkohol) ausgewählt wird/werden.

## Claims

1. Cosmetic preparations, **characterized in that** they are in the form of an O/W emulsion and comprise an active ingredient combination of polyhexamethylene biguanide hydrochloride and distearyldimethylammonium chloride.

2. Cosmetic preparations according to Claim 1, **characterized in that** they comprise active ingredient combinations of polyhexamethylene biguanide hydrochloride and distearyldimethyl ammonium chloride in a concentration range of 0.0005 to 50.0 weight %, more particularly 0.01 to 20.0 weight %, based on the total weight of the composition.

3. Cosmetic preparations according to Claim 1 and/or 2, **characterized in that** the active ingredients polyhexamethylene biguanide hydrochloride and distearyldimethylammonium chloride are included in a concentration range of 0.02 to 10.0 weight %, preferably 0.02 to 5.0 weight % and with particular preference 0.5 to 3.0 weight %, based on the total weight of the composition.

4. Cosmetic preparations according to one or more of the preceding claims, **characterized in that** the cosmetic preparations comprise one or more O/W emulsifiers.

5. Cosmetic preparations according to one or more of the preceding claims, **characterized in that** the cosmetic preparations comprise one or more co-emulsifiers.

6. Cosmetic preparations according to Claim 4, **characterized in that** the O/W emulsifier or emulsifiers is or are selected from the group of polyethoxylated and/or polypropoxylated emulsifiers.

7. Cosmetic preparations according to Claim 5, **characterized in that** the co-emulsifier or co-emulsifiers is or are selected from the group of fatty alcohols having 14 to 22 carbon atoms.

8. Cosmetic preparations according to Claim 5 and/or 7, **characterized in that** the co-emulsifier or co-emulsifiers is or are selected from the group of fatty alcohols having 16 to 18 carbon atoms such as stearyl alcohol, cetyl alcohol, cetyl stearyl alcohol (cetearyl alcohol).

## Revendications

1. Préparations cosmétiques, **caractérisées en ce qu'**elles se trouvent sous forme d'une émulsion H/E et contiennent une combinaison de substances actives de chlorhydrate de polyhexaméthylène-biguanide et de chlorure de distéaryldiméthylammonium.

2. Préparations cosmétiques selon la revendication 1, **caractérisées en ce qu'**elles contiennent des combinaisons de substances actives de chlorhydrate de polyhexaméthylène-biguanide et de chlorure de distéaryldiméthylammonium dans une plage de concentration de 0,0005 à 50,0% en poids, en particulier de 0,01 à 20,0% en poids, par rapport au poids total de la composition.

3. Préparations cosmétiques selon la revendication 1 et/ou 2, **caractérisées en ce que** les substances actives, le chlorhydrate de polyhexaméthylène-biguanide et le chlorure de distéaryldiméthylammonium, sont contenues dans une plage de concentration de 0,02 à 10,0% en poids, de préférence de 0,02 à 5,0% en poids et de manière particulièrement préférée de 0,5 à 3,0% en poids, par rapport au poids total de la composition.

4. Préparations cosmétiques selon l'une ou plusieurs des revendications précédentes, **caractérisées en ce que** les préparations cosmétiques contiennent un ou plusieurs émulsifiants H/E.

5. Préparations cosmétiques selon l'une ou plusieurs des revendications précédentes, **caractérisées en ce que** les préparations cosmétiques contiennent un ou plusieurs co-émulsifiants.

6. Préparations cosmétiques selon la revendication 4, **caractérisées en ce que** le ou les émulsifiants H/E sont choisis dans le groupe des émulsifiants polyéthoxylés et/ou polypropoxylés.

7. Préparations cosmétiques selon la revendication 5, **caractérisées en ce que** le ou les co-émulsifiants sont choisis dans le groupe des alcools gras comprenant 14 à 22 atomes de carbone.

8. Préparations cosmétiques selon la revendication 5 et/ou 7, **caractérisées en ce que** le ou les co-émulsifiants sont choisis dans le groupe des alcools gras comprenant 16 à 18 atomes de carbone, tels que l'alcool stéarylique, cétylique, cétylstéarylique (alcool cétéarylique).
